(19) Europäisches Patentamt
European Patent Office
Office européen des brevets

(11) Veröffentlichungsnummer: **0 219 747 B1**

# (12) EUROPÄISCHE PATENTSCHRIFT

(45) Veröffentlichungstag der Patentschrift: **16.05.90**

(51) Int. Cl.⁵: **C07D 471/04**, A61K 31/44 // (C07D471/04, 235:00, 221:00)

(21) Anmeldenummer: **86113715.6**

(22) Anmeldetag: **03.10.86**

(54) **Neue 2-(2-Thienyl)-imidazo(4,5-c)pyridinderivate und deren Salze, Verfahren zu ihrer Herstellung und diese enthaltende pharmazeutische Präparate.**

(30) Priorität: **09.10.85 AT 2919/85**

(43) Veröffentlichungstag der Anmeldung: **29.04.87 Patentblatt 87/18**

(45) Bekanntmachung des Hinweises auf die Patenterteilung: **16.05.90 Patentblatt 90/20**

(84) Benannte Vertragsstaaten: **AT BE CH DE ES FR GB GR IT LI LU NL SE**

(56) Entgegenhaltungen:
**EP-A- 0 072 926**
**EP-A- 0 145 252**
**EP-A- 0 148 742**
**EP-A- 0 155 094**

**Die Akte enthält technische Angaben, die nach dem Eingang der Anmeldung eingereicht wurden und die nicht in dieser Patentschrift enthalten sind.**

(73) Patentinhaber: **CL PHARMA AKTIENGESELLSCHAFT, St. Peter-Strasse 25, A-4021 Linz(AT)**

(72) Erfinder: **Binder, Dieter, Dr., Sieveringerstrasse 207, A-1190 Wien(AT)**
Erfinder: **Rovenszky, Franz, Dr., Dipl.-Ing., Lagerhausstrasse 5/8, A-2460 Bruck a.d. Leitha(AT)**

(74) Vertreter: **Kunz, Ekkehard, Dr., Chemie Holding AG Patentwesen St. Peter-Strasse 25, A-4021 Linz(AT)**

EP 0 219 747 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2-(2-Thienyl)-imidazo(4,5-c)pyridinderivate der allgemeinen Formel I des Formelblattes, worin
$R_1$ niederes Alkyl
$R_2$ Wasserstoff oder Methyl und
n 0 oder 1 bedeuten,
und die pharmazeutisch verwendbaren Säureadditionssalze von Verbindungen der allgemeinen Formel I, ein Verfahren zu ihrer Herstellung, pharmazeutische Präparate, die diese Verbindungen enthalten, sowie ihre Verwendung in Arzneimitteln.

Der in dieser Beschreibung verwendete Ausdruck "niederes Alkyl" bezeichnet geradkettige oder verzweigte Kohlenwasserstoffreste mit 1 bis 4 Kohlenstoffatomen, wie z.B. Methyl, Ethyl, Propyl, Isopropyl, Butyl, Isobutyl, sek. Butyl, tert. Butyl.

In einer bevorzugten Klasse von Verbindungen der allgemeinen Formel I bedeutet $R_1$ Methyl oder Ethyl, wobei $R_1$ in der Bedeutung von Methyl besonders bevorzugt ist.

Besonders bevorzugte Einzelverbindungen mit der allgemeinen Formel I sind 2-(3-Methoxy-5-methylthio-2-thienyl)-1H-imidazo(4,5-c)pyridin, 2-(3-Methoxy-5-methylsulfinyl-2-thienyl)-1H-imidazo(4,5-c)pyridin und die Hydrochloride dieser Verbindungen.

Die 2-(2-Thienyl)-imidazo(4,5-c)pyridinderivate der allgemeinen Formel I und deren Salze werden erfindungsgemäß nach an sich bekannten Methoden, vorzugsweise dadurch hergestellt, daß man

a) ein Thiophencarbonsäurederivat der allgemeinen Formel II des Formelblattes, worin $R_1$ die in Formel I genannte Bedeutung besitzt, oder ein Salz davon durch Umsetzung mit einem anorganischen Säurechlorid in ein Thiophencarbonsäurechlorid der allgemeinen Formel III des Formelblattes, worin $R_1$ die in Formel I angegebene Bedeutung besitzt, verwandelt, dieses mit einem Diaminopyridin der allgemeinen Formel IV des Formelblattes, worin $R_2$ wie in Formel I definiert ist, zu einem Isomerengemisch, bestehend aus den Amiden der allgemeinen Formel V des Formelblattes, worin $R_1$ und $R_2$ wie in Formel I definiert sind, eines der Symbole A und B das =N-Atom und das jeweils andere ein =CH-Ringglied des Pyridinrings bedeutet, umsetzt, die freien Basen im erhaltenen Isomerengemisch in die Hydrochloride überführt und diese mit wasserentziehenden Mitteln unter Bildung des Imidazolrings cyclisiert, worauf man

b) gegebenenfalls eine so erhaltene Verbindung der allgemeinen Formel I, worin n=0 bedeutet, durch Behandeln mit organischen Persäuren oder Wasserstoffperoxid zu einer Verbindung der allgemeinen Formel I umsetzt, in der n die Zahl 1 bedeutet, und

c) erwünschtenfalls eine in Verfahrensschritt a) oder b) erhaltene Verbindung der allgemeinen Formel I oder ein pharmazeutisch nicht verwendbares Salz davon mit anorganischen oder organischen Säuren in ein pharmazeutisch verwendbares Additionssalz überführt.

Die Herstellung der Thiophencarbonsäurechloride der allgemeinen Formel III in Verfahrensschritt a) erfolgt zweckmäßigerweise durch Umsetzen einer Thiophencarbonsäure der allgemeinen Formel II mit einem anorganischen Säurechlorid, z.B. Thionyl-, Phosphoroxy-, Phosphortri- oder Phosphorpentachlorid, bei niedrigen Temperaturen, wobei die Umsetzung mit Thionylchlorid bei Temperaturen um 0° C besonders bevorzugt ist.

Ein bei der obigen Umsetzung erhaltenes Thiophencarbonsäurechlorid der allgemeinen Formel III wird anschließend erfindungsgemäß mit einem 3,4-Diaminopyridin der allgemeinen Formel IV unter für die Amidbildung üblichen Reaktionsbedingungen umgesetzt, wobei ein Isomerengemisch, welches die durch Formel V dargestellten Amide enthält, entsteht. Die Umsetzung von Verbindungen der Formel III mit jenen der Formel IV erfolgt zweckmäßigerweise in einem geeigneten organischen Lösungsmittel, bevorzugt in Pyridin, unter Zusatz einer starken Base, wobei sich für diesen Zweck die Verwendung von Triethylamin als besonders vorteilhaft erwiesen hat. Die Herstellung der Amide der Formel V kann einfachheitshalber bei Raumtemperatur erfolgen, jedoch sind Temperaturen, die knapp darüber oder darunter liegen, an sich in gleicher Weise für die Umsetzung geeignet.

Die bei dieser Umsetzung im Isomerengemisch in Form der freien Basen erhaltenen Verbindungen der allgemeinen Formel V werden vor der Cyclisierung in die Hydrochloride übergeführt, was zweckmäßigerweise durch Einleiten von HCl-Gas in eine Lösung der freien Basen in einem inerten organischen Lösungsmittel und anschließende Abscheidung der Hydrochloride, beispielsweise durch Zugabe von trockenem Diethylether, geschehen kann, und dann gemäß Verfahrensschritt a) zu Verbindungen der allgemeinen Formel I, worin n = 0 bedeutet, durch Behandeln mit einem wasserentziehenden Mittel unter Bildung des Imidazolrings cyclisiert.

Die Cyclisierung kann durch direkte Behandlung des Gemisches der Hydrochloride der Verbindungen der allgemeinen Formel V oder vorteilhafterweise durch Behandeln von deren Suspensionen in einem geeigneten organischen Lösungs-oder Verdünnungsmittel erfolgen, wobei sich Suspensionen der Hydrochloride in Pyridin als besonders geeignet erwiesen haben. Als wasserentziehende Mittel kommen dabei die für solche Cyclisierungen üblicherweise verwendeten Reagenzien in Frage, beispielsweise Phosphoroxy-, Phosphorpenta- oder Thionylchlorid und dergleichen.

Das wasserentziehende Reagens kann hiezu in äquivalenten Mengen oder vorteilhafterweise in einem Überschuß, beispielsweise in Mengen von 1.1 bis 5 Mol pro Mol des Gemisches der Formel V eingesetzt werden. Als besonders vorteilhafte Cyclisierungsmethode zur Herstellung der erfindungsgemäßen Verbindungen hat sich die langsame Zugabe eines 1.5 bis 3-fachen molaren Überschusses an Phosphoroxychlorid zu einer Suspension der Hydrochloride von Amiden der allgemeinen Formel V bei Raumtemperatur oder Temperaturen, die knapp darunter oder darüber liegen, herausgestellt.

Gemäß Verfahrensschritt b) können die Sulfinylverbindungen, bei denen n in der allgemeinen Formel I die Zahl 1 bedeutet, ausgehend von den nach Verfahrensschritt a) erhaltenen Methylmercaptoverbindungen der allgemeinen Formel I mit der Bedeutung für n = 0 durch partielle Oxidation mit geeigneten Oxidationsmitteln erhalten werden. Als solche Oxidationsmittel werden vorzugsweise etwa äquivalente Mengen einer organischen Persäure, wie Peressigsäure, Perbenzoesäure, m-Chlorperbenzoesäure und dergleichen, in einem reaktionsinerten Lösungsmittel, z.B. Methylenchlorid oder Chloroform, zweckmäßigerweise bei Temperaturen zwischen -5° C und -50° C, vorzugsweise bei etwa 0° C, oder etwa äquivalente Mengen 30 %-iges Wasserstoffperoxid in Eisessig, vorteilhafterweise bei Raumtemperatur, verwendet.

Die nach Verfahrensschritt a) oder b) erhaltenen Verbindungen der allgemeinen Formel I haben stark basische Eigenschaften. Man kann sie gemäß Verfahrensschritt c) mit anorganischen oder organischen Säuren auf übliche Weise leicht in kristalline, pharmazeutisch verwendbare Säureadditionssalze überführen, die sich, wie z.B. die Hydrochloride, gut durch Umkristallisieren reinigen lassen. Dazu löst man die rohe Base zweckmäßigerweise in einem geeigneten Lösungsmittel, z.B. einem niederen aliphatischen Alkohol, gibt eine äquivalente Menge der gewünschten Säure zu, fällt die gebildeten Salze aus oder dampft im Vakuum das Lösungsmittel ab und kristallisiert den Rückstand aus Methanol oder Ethanol, gegebenenfalls unter Zusatz von Ether oder Aceton, um.

Geeignete Beispiele für derartige pharmazeutisch verwendbare Salze von Verbindungen der Formel I sind neben dem Salz der Salzsäure beispielsweie die Salze der Schwefelsäure, der Salpetersäure, von Sulfonsäuren, der Benzoesäure, der Maleinsäure, der Weinsäure, der Zitronensäure und ähnliche, zur Herstellung von pharmazeutisch verwendbaren Salzen bekannter Verbindungen bereits eingesetzte Säuren.

Die für das Verfahren zur Herstellung der erfindungsgemäßen Verbindungen als Ausgangsmaterialien verwendeten Thiophencarbonsäurederivate der allgemeinen Formel II sind literaturbekannt (EP-A-0148742) oder können ausgehend von bekannten Verbindungen in an sich bekannter Weise hergestellt werden. Die Diaminopyridine der allgemeinen Formel IV sind ebenfalls bekannt und unter anderem im Handel erhältlich (z.B. von Fluka AG, Buchs, Schweiz).

Die neuen Verbindungen der allgemeinen Formel I und in gleicher Weise ihre pharmazeutisch verwendbaren Säureadditionssalze zeigen sowohl in vitro als auch in vivo im Tierversuch äußerst wertvolle pharmakologische Eigenschaften.

Insbesondere haben sie eine stark positiv inotrope Wirkung auf das Herz und können daher infolge ihrer cardiotonischen Eigenschaften in der Humanmedizin zur Behandlung und Prophylaxe von Herz- und Kreislauferkrankungen, insbesondere zur Behandlung der myocardialen Insuffizienz und deren pathologischer Folgeerscheinungen, eingesetzt werden.

Zur Feststellung dieser pharmakologischen Eigenschaften wurden erfindungsgemäße Verbindungen folgenden, allgemein anerkannten pharmakologischen Untersuchungen unterzogen:

1) Positiv inotrope Wirkung am isolierten Meerschweinchen-Vorhof

Die Wirkung von Verbindungen der allgemeinen Formel I auf die Frequenz und Kontraktionskraft von frisch präparierten, isolierten Meerschweinchen-Vorhöfen wurde geprüft. Dazu wurden die Testsubstanzen in Konzentrationen im Bereich von $10^{-7}$ bis $3.10^{-4}$ Mol/l untersucht, wobei in einer ersten Versuchsreihe die Vorhöfe unbehandelter Tiere und in einer zweiten Versuchsreihe jene von reserpinisierten Tieren verwendet wurden.

Ergebnis:

In diesem Standardtest bewirken erfindungsgemäße Verbindungen eine signifikante, dosisabhängige Steigerung der Kontraktionskraft isolierter, elektrisch gereizter, linker Meerschweinchen-Vorhöfe. Beispielsweise bewirkt die Verbindung der allgemeinen Formel I, 2-(3-Methoxy-5-methylsulfinyl-2-thienyl)-1H-imidazo(4,5-c)pyridin-hydrochlorid in einer Konzentration von $1.2 \cdot 10^{-4}$ Mol/l eine 50 %-ige Steigerung der Kontraktionskraft. Die Wirkung auf die Kontraktionskraft ist damit qualitativ vergleichbar mit jener des bekannten Herzglykosids Quabain, i.e. 3-((6-Desoxy-alpha-L-mannopyranosyl)oxy)1, 5, 11 alpha, 14, 19-pentahydroxy-card-20(22)-enolid.

Gegenüber Quabain haben die erfindungsgemäßen Verbindungen, wie 2-(3-Methoxy-5-methylsulfinyl-2-thienyl)-1H-imidazo(4,5-c)pyridin-hydrochlorid, den Vorteil, daß auch im oberen untersuchten Konzentrationsbereich keine therapeutisch unerwünschte Frequenzsteigerung am spontan schlagenden rechten Meerschweinchen-Vorhof eintritt.

Reserpinvorbehandlung beeinträchtigt die Wirkung der untersuchten Substanzen nur geringfügig, sodaß für die erfindungsgemäßen Verbindungen ein direkter Wirkungsmechanismus angenommen werden kann. Demnach zeigen Verbindungen der allgemeinen Formel I am isolierten Vorhof des Meerschweinchens eine deutliche, direkte positiv inotrope Wirkung bei nur sehr schwach ausgeprägter positiv chronotroper Wirkung.

B) Untersuchungen am narkotisierten Hund

Mit alpha-Chloralose (1,2-O-(2,2,2-Trichlorethyliden)-alpha-D-glucofuranose) narkotisierten Beagle-Hunden wurden erfindungsgemäße Verbindungen in Dosen von 3 bis 3000 µg/kg kumulativ in die V.jugularis injiziert. Nach der Applikation wurde die Wirkung auf den linksventrikulären Druck (LV dp/dt), den Femoralisdruck und -fluß, das Herzzeitvolumen (HZV) und die Herzfrequenz untersucht.

Ergebnis:

In diesen Untersuchungen bewirken die erfindungsgemäßen Verbindungen einerseits einen signifikanten, dosisabhängigen Anstieg des linksvertikulären Drucks und des Herzzeitvolumens, andererseits einen Abfall des linksvertikulären enddiastolischen Drucks und des peripheren diastolischen Blutdrucks.

So führt beispielsweise 2-(3-Methoxy-5-methylsulfinyl-2-thienyl)-1H-imidazo(4,5-c)pyridin-hydrochlorid schon bei einer Dosis von 300 µg/kg zu einem starken Anstieg des linksvertikulären Drucks (LV dp/dt = + 113 %) ohne unerünschte Beeinflußung der Herzfrequenz. Die Substanz zeigt bei dieser Dosis eine signifikante, stark positiv inotrope Wirkung am narkotisierten Hund. Eine deutliche Herzfrequenzsteigerung tritt erst bei Dosen von 1 bis 3 mg/kg i.v., die weit außerhalb des therapeutischen Anwendungsbereiches liegen, auf.

Als Substanzen mit positiv inotroper Wirkung werden bis jetzt hauptsächlich Herzglykoside oder sympathomimetische Amine verabreicht. Herzglykoside rufen in vielen Fällen Nebenwirkungen, wie gefährliche Herzrhythmusstörungen hervor und müssen infolge ihrer hohen Toxizität mit Vorsicht angewandt werden. Sympathomimetische Amine haben unter anderem wegen ihrer positiv chronotropen Nebenwirkung, arrhythmogenen Eigenschaften und oralen Unwirksamkeit nur begrenzte Anwendungsmöglichkeiten. Die erfindungsgemäßen Substanzen stellen somit wegen ihrer ausgeprägten und zugleich sehr spezifischen positiv inotropen Wirkung eine interessante Klasse von neuen Wirkstoffen dar, die die bisher angewandten Präparate zur Behandlung der myocardialen Insuffizienz mit großem Vorteil für den Patienten ersetzen könnten.

Aufgrund ihrer günstigen Eigenschaften können die Verbindungen der allgemeinen Formel I und deren Salze als Heilmittel, z.B. in Form pharmazeutischer Präparate Verwendung finden, welche die erfindungsgemäßen Verbindungen in Mischung mit einem für enterale oder parenterale Applikation geeigneten, pharmazeutischen, organischen oder anorganischen Trägermaterial, beispielsweise Wasser, Gelatine, Gummi arabicum, Milchzucker, Stärke, Magnesiumstearat, Talk, pflanzliche Öle, pflanzliche oder tierische Fette, Polyalkylenglykole, Vaseline und dergleichen enthalten. Die pharmazeutischen Präparate können in fester Form, z.B. als Tabletten, Dragees, Suppositorien, Kapseln oder in flüssiger Form, z.B. als Lösungen, Suspensionen oder Emulsionen vorliegen. Gegebenenfalls sind sie sterilisiert und/oder enthalten Hilfsstoffe, wie KonservierungsStabilisierungs- oder Emulgiermittel, Salze zur Veränderung des osmotischen Druckes. Insbesondere können sie auch in Kombination mit anderen therapeutisch wertvollen Stoffen verabreicht werden.

Die folgenden Beispiele erläutern die Erfindung näher

Beispiel 1:

<u>2-(3-Methoxy-5-methylthio-2-thienyl)-1H-imidazo(4,5-c)pyridin</u>

4,0 g (19,6 mmol) 3-Methoxy-5-methylthio-2-thiophencarbonsäure werden bei 0°C in 40 ml $SOCl_2$ eingerührt, 20 Min. gerührt und bei Raumtemperatur das überschüssige Lösungsmittel im Vakuum abgedampft.

Das rohe Säurechlorid (4,3 g, Fp = 98 - 101 °C, Diisopropylether) wird in 25 ml trockenem Benzol gelöst und zu einer Mischung aus 2,1 g (19,6 mmol) 3,4-Diaminopyridin 14 ml absolutem Pyridin und 10 ml Triethylamin innerhalb von 10 Min. bei 20 °C zugetropft. Es wird noch 90 Min. bei Raumtemperatur gerührt.

Das Reaktionsgemisch wird weitgehend eingedampft, mit 50 ml Wasser verdünnt, mit 2n HCl auf pH4 angesäuert, mit $NaHCO_3$ auf pH7 gebracht und dreimal mit insgesamt 200 ml Ethylacetat extrahiert. Die vereinigten organischen Phasen werden über Natriumsulfat getrocknet, filtriert und auf 50 ml eingeengt. In die verbleibende Lösung wird bei einer Temperatur unter 30 °C bis zur Sättigung HCl-Gas eingeleitet und anschließend mit 250 ml trockenem Diethylether versetzt. Es wird von dem abgeschiedenen Öl dekantiert, dieses noch zweimal mit je 100 ml trockenem Ether gewaschen und im Vakuum vom restlichen Ether befreit.

Das Gemisch der Amide wird in 50 ml abs. Pyridin suspendiert und 5,76 g (37,6 mmol) $POCl_3$ unter Rühren innerhalb von 5 Min. bei 20 °C zugetropft. Es wird noch 90 Min. bei Raumtemperatur gerührt. Anschließend wird weitgehend eingedampft und der Rückstand zwischen 50 ml gesättigter $NaHCO_3$-Lösung und 50 ml Ethylacetat verteilt. die wäßrige Phase wird noch zweimal mit je 40 ml Ethylacetat extrahiert, die vereinigten organischen Phasen über Natriumsulfat getrocknet und eingedampft. Der Rückstand (2,8 g) wird in 150 ml Methanol aufgenommen, mit überschüssiger methanolischer Salzsäure versetzt, nach Zusatz von Aktivkohle heiß filtriert, die Lösung auf ca. 50 ml eigeengt, abgekühlt und das ausgefallene gelbe Hydrochlorid abgesaugt, mit wenig kaltem Methanol gewaschen und im Vakuum getrocknet (1,6 g).

Zur Herstellung der freien Base werden 1,6 g (5,10 mmol) des Hydrochlorids in 12 ml Methanol suspendiert, mit überschüssigem wäßrigen Ammoniak versetzt und durch Zusatz von 40 ml Wasser gefällt. Es wird abgesaugt, mit Wasser gewaschen und bei 60 °C/20 mbar getrocknet.
Ausbeute: 1,27 g
Fp. = 165 - 167 °C (2-Butanon/$Et_2O$ = 3 : 1)

Beispiel 2:

2-(3-Methoxy-5-methylsulfinyl-2-thienyl)-1H-imidazo(4,5-c-)pyridin

0,50 g (1,80 mmol) 2-(3-Methoxy-5-methylthio-2-thienyl)-1H-imidazo(4,5-c)pyridin werden in 30 ml Chloroform gelöst, die Lösung auf -10 °C gekühlt und innerhalb von 10 Min. eine Lösung von 0,40 g (1,98 mmol) 85 proz. 3-Chlorperbenzoesäure in 10 ml Chloroform bei dieser Temperatur unter Rühren zugetropft. Es wird noch 10 Min. bei -10 °C gerührt, zweimal mit je 8 ml gesättigter $NaHCO_3$-Lösung extrahiert, die organische Phase über Natriumsulfat getrocknet und eingedampft.
Ausbeute: 0,46 g (87 %)
Fp. = 235 °C (Methanol)

Beispiel 3:

2-(3-Methoxy-5-methylthio-2-thienyl)-1H-imidazo(4,5-c)pyridin-hydrochlorid

1,0 g (3,6 mmol) 2-(3-Methoxy-5-methylthio-2-thienyl)-1H-imidazo-(4,5-c)pyridin werden in 25 ml Methanol suspendiert und mit 7 ml 1n methanolischer HCl versetzt. Es wird im Vakuum zur Trockene eingedampft und der Rückstand aus Methanol umkristallisiert.
Ausbeute: 1,04 g (92 %)
Fp. = 234 - 236 °C (Zers.; Methanol)

Beispiel 4:

2-(3-Methoxy-5-methylsulfinyl-2-thienyl)-1H-imidazo(4,5-c)pyridin-hydrochlorid

0,4 g (1,36 mmol) 2-(3-Methoxy-5-methylsulfinyl-2-thienyl)-1H-imidazo(4,5-c)pyridin werden in 13 ml Methanol suspendiert und mit 3 ml 1n methanolischer HCl versetzt. Es wird zur Trockene eingedampft und der Rückstand aus Methanol/Aceton umkristallisiert.
Ausbeute: 0,37 g (83 %)
Fp. = 204 - 207 °C (Methanol/Aceton)

**Patentansprüche für die Vertragsstaaten: BE, CH, DE, FR, GB, IT, LI, LU, NL, SE**

1. 2-(2-Thienyl)-imidazo(4,5-c)pyridinderivate der allgemeinen Formel I

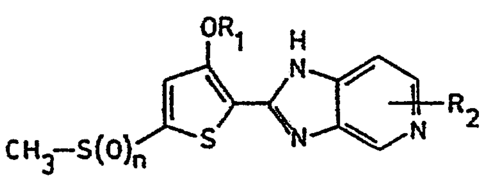

I

worin
$R_1$ $C_1$–$C_4$ Alkyl
$R_2$ Wasserstoff oder Methyl und
n 0 oder 1 bedeuten,
und die pharmazeutisch verwendbaren Säureadditionssalze von Verbindungen der allgemeinen Formel I.
2. Verbindungen der in Anspruch 1 definierten allgemeinen Formel I, worin $R_1$ Methyl oder Ethyl bedeutet.
3. Die Verbindungen nach Anspruch 1, 2-(3-Methoxy-5-methylthio-2-thienyl)-1H-imidazo(4,5-c)pyridin und dessen Hydrochlorid.
4. Die Verbindungen nach Anspruch 1, 2-(3-Methoxy-5-methylsulfinyl-2-thienyl)-1H-imidazo(4,5-c)pyridin und dessen Hydrochlorid.
5. Verfahren zur Herstellung von Verbindungen der in Anspruch 1 definierten allgemeinen Formel I, dadurch gekennzeichnet, daß man
a) ein Thiophencarbonsäurederivat der allgemeinen Formel II

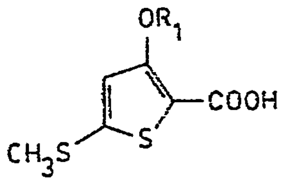

II

worin $R_1$ die in Formel I genannte Bedeutung besitzt, oder ein Salz davon durch Umsetzung mit einem anorganischen Säurechlorid in ein Thiophencarbonsäurechlorid der allgemeinen Formel III

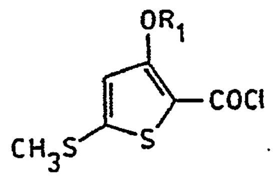

III

worin $R_1$ die in Formel I angegebene Bedeutung besitzt, verwandelt, dieses mit einem Diaminopyridin der allgemeinen Formel IV

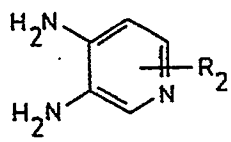

IV

worin $R_2$ wie in Formel I definiert ist, zu einem Isomerengemisch, bestehend aus Amiden der allgemeinen Formel V

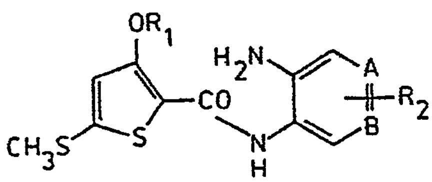

V

worin $R_1$ und $R_2$ wie in Formel I definiert sind, eines der Symbole A und B das =N-Atom und das jeweils andere ein =CH-Ringglied des Pyridinrings bedeutet, umsetzt, die freien Basen im erhaltenen Isomerengemisch in die Hydrochloride überführt und diese mit wasserentziehenden Mitteln unter Bildung des Imidazolrings cyclisiert, worauf man

b) gegebenenfalls eine so erhaltene Verbindung der allgemeinen Formel I, worin n = 0 bedeutet, durch Behandeln mit organischen Persäuren oder Wasserstoffperoxid zu einer Verbindung der allgemeinen Formel I umsetzt, in der n die Zahl 1 bedeutet, und

c) erwünschtenfalls eine in Verfahrensschritt a) oder b) erhaltene Verbindung der allgemeinen Formel I oder ein pharmazeutisch nicht verwendbares Salz davon mit anorganischen oder organischen Säuren in ein pharmazeutisch verwendbares Additionssalz überführt.

6. Pharmazeutische Präparate, enthaltend Verbindungen der allgemeinen Formel I nach Anspruch 1 oder deren Säureadditionssalze in Kombination mit in der Galenik üblichen Hilfs- und Trägerstoffen.

7. Verbindungen der allgemeinen Formel I nach Anspruch 1 oder deren Säureadditionssalze als Wirkstoffe für pharmazeutische Präparate mit positiv inotroper Wirkung.

8. Verbindungen nach Anspruch 1 oder deren Säureadditionssalze zur Verwendung als Wirkstoffe für Arzneimittel zur Behandlung oder Prophylaxe von Herz- und Kreislauferkrankungen, insbesondere der myocardialen Insuffizienz.

**Patentansprüche für die Vertragsstaaten: AT, GR, ES**

1. Verfahren zur Herstellung neuer 2-(2-Thienyl)-imidazol(4,5-c)pyridinderivate der allgemeinen Formel I

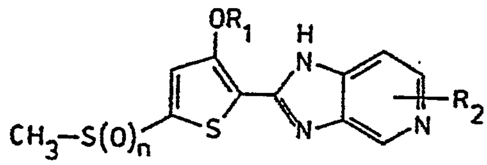

I

worin
$R_1$ $C_1$–$C_4$ Alkyl
$R_2$ Wasserstoff oder Methyl und
N 0 oder 1 bedeuten,
und von pharmazeutisch verwendbaren Säureadditionssalzen von Verbindungen der allgemeinen Formel I, dadurch gekennzeichnet, daß man

a) ein Thiophencarbonsäurederivat der allgemeinen Formel II

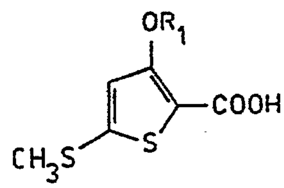

II

worin $R_1$ die in Formel I genannte Bedeutung besitzt, oder ein Salz davon durch Umsetzung mit einem anorganischen Säurechlorid in ein Thiophencarbonsäurechlorid der allgemeinen Formel III

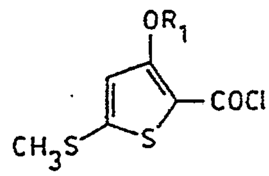

III

worin $R_1$ die in Formel I angegebene Bedeutung besitzt, verwandelt, dieses mit einem Diaminopyridin der allgemeinen Formel IV

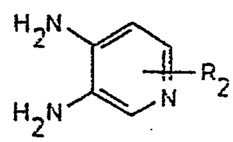

IV

worin $R_2$ wie in Formel I definiert ist, zu einem Isomerengemisch, bestehend aus Amiden der allgemeinen Formel V

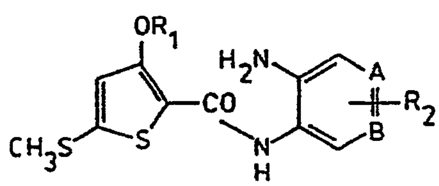

V

worin $R_1$ und $R_2$ wie in Formel I definiert sind, eines der Symbole A und B das =N-Atom und das jeweils andere ein =CH-Ringglied des Pyridinrings bedeutet, umsetzt, die freien Basen im erhaltenen Isomerengemisch in die Hydrochloride überführt und diese mit wasserentziehenden Mitteln unter Bildung des Imidazolrings cyclisiert, worauf man

b) gegebenenfalls eine so erhaltene Verbindung der allgemeinen Formel I, worin n = 0 bedeutet, durch Behandeln mit organischen Persäuren oder Wasserstoffperoxid zu einer Verbindung der allgemeinen Formel I umsetzt, in der n die Zahl 1 bedeutet, und

c) erwünschtenfalls eine in Verfahrensschritt a) oder b) erhaltene Verbindung der allgemeinen Formel I oder ein pharmazeutisch nicht verwendbares Salz davon mit anorganischen oder organischen Säuren in ein pharmazeutisch verwendbares Additionssalz überführt.

2. Verfahren nach Anspruch 1 zur Herstellung von Verbindungen der allgemeinen Formel I, worin $R_1$ Methyl bedeutet, $R_2$ und n wie in Anspruch 1 definiert sind, dadurch gekennzeichnet, daß man als Ausgangsmaterial der Formel II 3-Methoxy-5-methylthio-2-thiophencarbonsäure einsetzt.

3. Verfahren nach einem der Ansprüche 1 oder 2 zur Herstellung von 2-(3-Methoxy-5-methylthio-2-thienyl)-1H-imidazo(4,5-c)pyridin und dessen Hydrochlorid, dadurch gekennzeichnet, daß man als Ausgangsmaterial der Formel II 3-Methoxy-5-methylthio-2-thiophencarbonsäure und als Ausgangsmaterial der Formel IV, 2,4-Diaminopyridin einsetzt und erwünschtenfalls eine erhaltene freie Base oder ein nicht verwendbares Säureadditionssalz davon in das Hydrochlorid überführt.

4. Verfahren nach einem der Ansprüche 1 bis 3, dadurch gekennzeichnet, daß man in Verfahrensschritt a) eine Thiophencarbonsäure der allgemeinen Formel II durch Behandeln mit Thionylchlorid bei einer Temperatur von 0°C in das Säurechlorid der allgemeinen Formel III umwandelt.

5. Verfahren nach einem der Ansprüche 1 bis 4, dadurch gekennzeichnet, daß man in Verfahrensschritt a) die Umsetzung einer Verbindung der allgemeinen Formel III mit jener der allgemeinen Formel IV in Pyridin unter Zusatz von Triethylamin bei Raumtemperatur durchführt.

6. Verfahren nach einem der Ansprüche 1 bis 5, dadurch gekennzeichnet, daß man in Verfahrensschritt a) ein Isomerengemisch der allgemeinen Formel V durch Zugabe eines 1,5 bis 3-fachen molaren Überschusses an Phosphoroxychlorid bei Raumtemperatur cyclisiert.

7. Verfahren nach einem der Ansprüche 1 oder 2 zur Herstellung von 2-(3-Methoxy-5-methylsulfinyl-2-thienyl)-1H-imidazo(4,5-c)pyridin und dessen Hydrochlorid, dadurch gekennzeichnet, daß man 2-(3-Methoxy-5-methylthio-2-thienyl)-1H-imidazo(4,5-c)pyridin mit einer organischen Persäure oder Wasserstoffperoxid behandelt und erwünschtenfalls eine erhaltene freie Base oder ein pharmazeutisch nicht verwendbares Salz davon in das Hydrochlorid überführt.

8. Verfahren nach einem der Ansprüche 1, 2 oder 7, dadurch gekennzeichnet, daß man die Oxidation in Stufe b) mit etwa äquivalenten Mengen 3-Chlorperbenzoesäure, Perbenzoesäure oder Peressigsäure durchführt.

9. Verfahren nach Anspruch 8, dadurch gekennzeichnet, daß man die Oxidation in Methylenchlorid oder Chloroform bei –5°C bis –50°C, vorzugsweise etwa 0°C durchführt.

10. Verfahren nach den Ansprüchen 1, 2 oder 7, dadurch gekennzeichnet, daß man die Oxidation mit etwa äquivalenten Mengen Wasserstoffperoxid durchführt.

11. Verfahren nach Anspruch 10, dadurch gekennzeichnet, daß man die Oxidation in 30% Wasserstoffperoxid in Eisessig bei Raumtemperatur durchführt.

**Claims for the Contracting States: BE, CH, DE, FR, GB, IT, LI, LU, NL, SE**

1. 2-(2-thienyl)-imidazo(4,5-c)pyridine derivatives of the general formula I

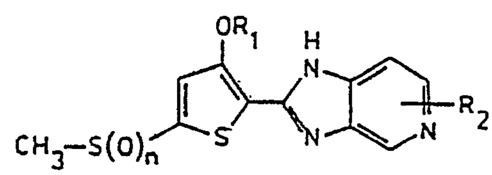

I.

wherein $R_1$ denotes $C_1$–$C_4$alkyl, $R_2$ denotes hydrogen or methyl and n denotes 0 or 1, and the pharmaceutically usable acid addition salts of compounds of the general formula I.

2. Compounds of the general formula I defined in claim 1, wherein $R_1$ denotes methyl or ethyl.

3. The compounds according to claim 1, 2-(3-methoxy-5-methylthio-2-thienyl)-1H-imidazo(4,5-c)pyridine and the hydrochloride thereof.

4. The compounds according to claim 1, 2-(3-methoxy-5-methylsulfinyl-2-thienyl)-1H-imidazo(4,5-c)pyridine and the hydrochloride thereof.

5. Process for the preparation of compounds of the general formula I defined in claim 1, characterized in that

a) a thiophenecarboxylic acid derivative of the general formula II

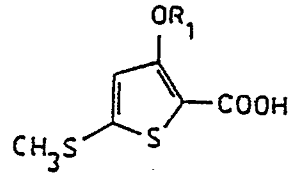

II

wherein $R_1$ has the meaning given in the case of formula I, or a salt thereof is converted into a thiophenecarboxylic acid chloride of the general formula III

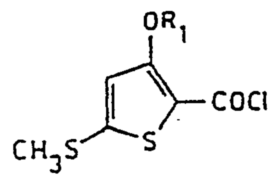 III

wherein $R_1$ has the meaning given in the case of formula I, by reaction with an inorganic acid chloride, this product is reacted with a diaminopyridine of the general formula IV

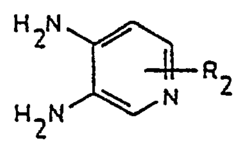 IV

wherein $R_2$ is as defined in formula I, to give an isomer mixture consisting of the amides of the general formula V

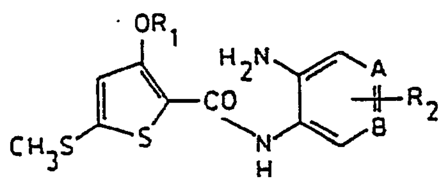 V

wherein $R_1$ and $R_2$ are as defined in formula I, one of the symbols A and B denotes the =N atom and the other in each case denotes a =CH ring member of the pyridine ring, the free bases in the resulting isomer mixture are converted into the hydrochlorides and these are cyclized with dehydrating agents to form the imidazole ring, after which
b) if appropriate, a resulting compound of the general formula I wherein n denotes 0 is converted into a compound of the general formula I in which n denotes the number 1 by treatment with organic peracids or hydrogen peroxide, and
c) if desired, a compound of the general formula I obtained in process step (a) or (b) or a salt thereof which cannot be used pharmaceutically is converted into a pharmaceutically usable addition salt with inorganic or organic acids.

6. Pharmaceutical products containing compounds of the general formula I according to claim 1 or acid addition salts thereof in combination with the auxiliaries and excipients customary in galenics.

7. Compounds of the general formula I according to claim 1 or acid addition salts thereof as active compounds for pharmaceutical products with a positively inotropic action.

8. Compounds according to claim 1 or acid addition salts thereof for use as active compounds for medicaments for the treatment or prophylaxis of diseases of the heart and circulation, in particular myocardial insufficiency.

**Claims for the Contracting States: AT, GR, ES**

1. Process for the preparation of new 2-(2-thienyl)-imidazo(4,5-c)pyridine derivatives of the general formula I

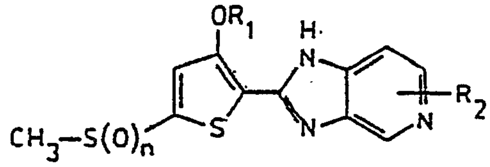 I.

wherein $R_1$ denotes $C_1$–$C_4$alkyl, $R_2$ denotes hydrogen or methyl and n denotes 0 or 1, and the pharmaceutically usable acid addition salts of compounds of the general formula I, characterized in that

a) a thiophenecarboxylic acid derivative of the general formula II

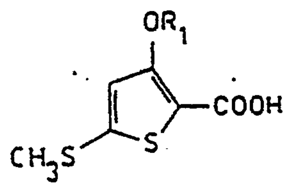

II

wherein $R_1$ has the meaning given in the case of formula I, or a salt thereof is converted into a thiophenecarboxylic acid chloride of the general formula III

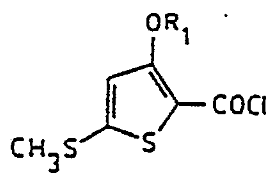

III

wherein $R_1$ has the meaning given in the case of formula I, by reaction with an inorganic acid chloride, this product is reacted with a diaminopyridine of the general formula IV

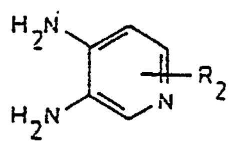

IV

wherein $R_2$ is as defined in formula I, to give an isomer mixture consisting of the amides of the general formula V

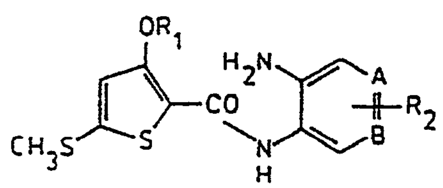

V

wherein $R_1$ and $R_2$ are as defined in formula I, one of the symbols A and B denotes the =N atom and the other in each case denotes a =CH ring member of the pyridine ring, the free bases in the resulting isomer mixture are converted into the hydrochlorides and these are cyclized with dehydrating agents to form the imidazole ring, after which

b) if appropriate, a resulting compound of the general formula I wherein n denotes 0 is converted into a compound of the general formula I in which n denotes the number 1 by treatment with organic peracids or hydrogen peroxide, and

c) if desired, a compound of the general formula I obtained in process step (a) or (b) or a salt thereof which cannot be used pharmaceutically is converted into a pharmaceutically usable addition salt with inorganic or organic acids.

2. Process according to claim 1 for the preparation of compounds of the general formula I, where $R_1$ denotes methyl, $R_2$ and n are as defined in claim 1, characterized in that 3-methoxy-5-methylthio-2-thiophenecarboxylic acid is employed as the starting material of the formula II.

3. Process according to one of claims 1 or 2 for the preparation of 2-(3-methoxy-5-methylthio-2-thienyl)-1H-imidazo[4,5-c]pyridine and its hydrochloride, characterized in that 3-methoxy-5-methylthio-2-thiophenecarboxylic acid is employed as the starting material of the formula II and 2,4-diaminopyridine is employed as the starting material of the formula IV and, if desired, a resulting free base or an acid addition salt thereof which cannot be used is converted into the hydrochloride.

4. Process according to one of claims 1 to 3, characterized in that, in process step a), a thiophenecarboxylic acid of the general formula II is converted into the acid chloride of the general formula III by treating with thionyl chloride at a temperature of 0°C.

5. Process according to one of claims 1 to 4, characterized in that, in process step a), the reaction of a compound of the general formula III with that of the general formula IV is carried out at room temperature in pyridine with the addition of triethylamine.

6. Process according to one of claims 1 to 5, characterized in that, in process step a), an isomer mixture of the general formula V is cyclised at room temperature by addition of a 1.5 to 3-fold molar excess of phosphorus oxychloride.

7. Process according to one of claims 1 or 2 for the preparation of 2-(3-methoxy-5-methylsulfinyl-2-thienyl)-1H-imidazo(4,5-c)pyridine and its hydrochloride, characterized in that 2-(3-methoxy-5-methylthio-2-thienyl)-1H-imidazo(4,5-c)pyridine is treated with an organic peracid or hydrogen peroxide and, if desired, a resulting free base or a salt thereof which cannot be used pharmaceutically is converted into the hydrochloride.

8. Process according to one of claims 1, 2 or 7, characterized in that the oxidation in step b) is carried out with approximately equivalent amounts of 3-chloroperbenzoic acid, perbenzoic acid or peracetic acid.

9. Process according to claim 8, characterized in that the oxidation is carried out in methylene chloride or chloroform at –5°C to –50°C, preferably about 0°C.

10. Process according to claims 1, 2 or 7, characterized in that the oxidation is carried out with approximately equivalent amounts of hydrogen peroxide.

11. Process according to claim 10, characterized in that the oxidation is carried out at room temperature in 30% hydrogen peroxide in glacial acetic acid.

**Revendications pour les Etats contractants: BE, CH, DE, FR, GB, IT, LI, LU, NL, SE**

1. Dérivés de la 2-(2-thiényl)-imidazo(4,5-c)pyridine de formule générale I

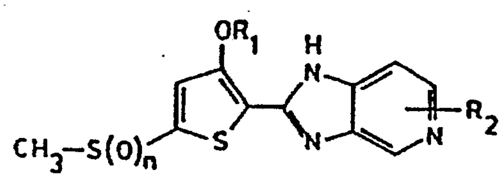

I

dans laquelle $R_1$ représente un radical alcoyle inférieur, $R_2$ représente un atome d'hydrogène ou le radical méthyle en n représente le nombre 0 ou 1, et les sels d'addition d'acides pharmaceutiquement acceptables des composés de formule générale I.

2. Composés de formule générale I définie dans la revendication 1, dans laquelle $R_1$ représente le radical méthyle ou éthyle.

3. Les composés selon la revendication 1, 2-(3-méthoxy-5-méthylthio-2-thiényl)-1H-imidazo(4,5-c)pyridine et son chlorhydrate.

4. Les composés selon la revendication 1, 2-(3-méthoxy-5-méthylsulfinyl-2-thiényl)-1H-imidazo(4,5-c)pyridine et son chlorhydrate.

5. Procédé pour la préparation des composés de formule générale I, définie dans la revendication 1, caractérisé en ce que

a) on convertit un dérivé d'acide thiophènecarboxylique de formule générale II

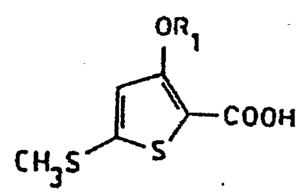

II

dans laquelle $R_1$ est défini comme spécifié dans la formule I, ou un sel de celui-ci par réaction avec un chlorure d'acide inorganique en un chlorure d'acide thiophènecarboxylique de formule générale III

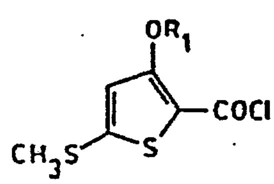

III

dans laquelle $R_1$ est défini comme spécifié dans la formule I, on fait réagir celui-ci avec une diaminopyridine de formule générale IV

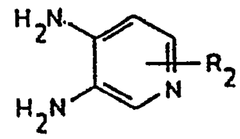

IV

dans laquelle $R_2$ est défini comme spécifié dans la formule I, en obtenant un mélange d'isomères, constitués par des amides de formule générale V

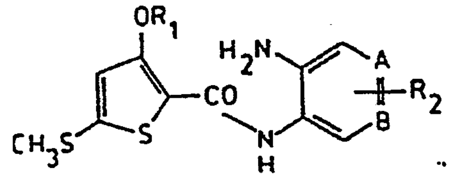

V

dans laquelle $R_1$ et $R_2$ sont définis comme spécifié dans la formule I, l'un des symboles A et B représente l'atome =N- et l'autre symbole représente respectivement un chaînon cyclique =CH- du noyau pyridine, on transforme les bases libres du mélange d'isomères obtenu en chlorhydrates et on cyclise ceux-ci avec des agents déshydratants avec formation du noyau imidazole, après quoi

b) on fait éventuellement réagir un composé ainsi obtenu de formule générale I, dans laquelle n = 0, par traitement avec des peracides organiques ou avec l'eau oxygénée en obtenant un composé de formule générale I, dans laquelle n représente le nombre 1, et

c) si on le désire, on transforme un composé de formule générale I ou un sel pharmaceutiquement non acceptable de celui-ci, obtenu dans le stade opératoire a) ou b), avec des acides inorganiques ou organiques en un sel d'addition pharmaceutiquement acceptable.

6. Compositions pharmaceutiques, contenant des composés de formule générale I selon la revendication 1 ou leurs sels d'addition d'acides en combinaison avec des adjuvants et véhicules courants en pharmacie galénique.

7. Composé de formule générale I selon la revendication 1 ou leurs sels d'addition d'acides en tant que substances actives pour compositions pharmaceutiques à activité inotrope positive.

8. Composés selon la revendication 1 ou leurs sels d'addition d'acides pour utilisation comme substances actives dans les médicaments pour le traitement ou la prophylaxie des maladies cardiaques et circulatoires, en particulier pour l'insuffisance myocardique.

**Revendications pour les Etats contractants: AT, GR, ES**

1. Procédé pour la préparation des nouveaux dérivés de la 2-(2-thiényl)-imidazo(4,5-c)pyridine de formule générale I

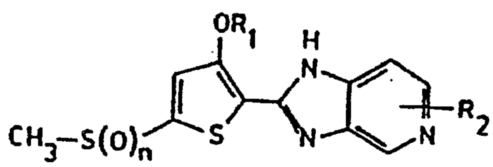

I

dans laquelle $R_1$ représente un radical alcoyle inférieur, $R_2$ représente un atome d'hydrogène ou le radical méthyle et n représente le nombre 0 ou 1, et des sels d'addition d'acides pharmaceutiquement acceptables des composés de formule générale I, caractérisé en ce que

a) on convertit un dérivé d'acide thiophènecarboxylique de formule générale II

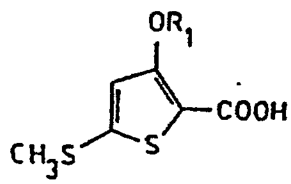

II

dans laquelle $R_1$ est défini comme spécifié dans la formule I, ou un sel de celui-ci par réaction avec un chlorure d'acide inorganique en un chlorure d'acide thiophènecarboxylique de formule générale III

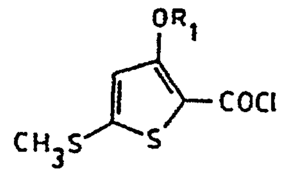

III

dans laquelle $R_1$ est défini comme spécifié dans la formule I, on fait réagir celui-ci avec une diaminopyridine de formule générale IV

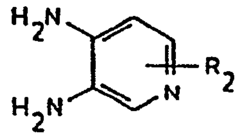

IV

dans laquelle $R_2$ est défini comme spécifié dans la formule I, en obtenant un mélange d'isomères, constitués par des amides de formule générale V

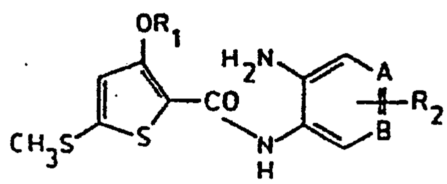

V

dans laquelle $R_1$ et $R_2$ sont définis comme spécifié dans la formule I, l'un des symboles A et B représente l'atome =N- et l'autre symbole représente respectivement un chaînon cyclique =CH- du noyau pyridine, on transforme les bases libres du mélange d'isomères obtenu en chlorhydrates et on cyclise ceux-ci avec des agents déshydratants avec formation du noyau imidazole, après quoi

b) on fait éventuellement réagir un composé ainsi obtenu de formule générale I, dans laquelle n = 0, par traitement avec des peracides organiques ou avec l'eau oxygénée en obtenant un composé de formule générale I, dans laquelle n représente le nombre 1, et

c) si on le désire, on transforme un composé de formule générale I ou un sel pharmaceutiquement non acceptable de celui-ci, obtenu dans le stade opératoire a) ou b), avec des acides inorganiques ou organiques en un sel d'addition pharmaceutiquement acceptable.

2. Procédé selon la revendication 1 pour la préparation des composés de formule générale I, dans laquelle $R_1$ représente le radical méthyle, tandis que $R_2$ et n sont définis comme spécifié dans la revendication 1, caractérisé en ce que l'on utilise comme substance de départ de formule II, l'acide 3-méthoxy-5-méthylthio-2-thiophènecarboxylique.

3. Procédé selon l'une des revendications 1 ou 2 pour la préparation de la 2-(3-méthoxy-5-méthylthio-2-thiényl)-1H-imidazo(4,5-c)pyridine et de son chlorhydrate, caractérisé en ce qu'on utilise comme substance de départ de formule II l'acide 3-méthoxy-5-méthylthio-2-thiophènecarboxylique et comme substance de départ de formule IV, la 2,4-diaminopyridine et en ce que si on le désire, on transforme une base libre obtenue ou un sel d'addition d'acide non acceptable de celle-ci, en chlorhydrate.

4. Procédé selon l'une des revendications 1 à 3, caractérisé en ce que dans le stade opératoire a), on convertit un acide thiophènecarboxylique de formule générale II par traitement avec le chlorure de thionyle à une température de 0°C en chlorure d'acide de formule générale III.

5. Procédé selon l'une des revendications 1 à 4, caractérisé en ce que dans le stade opératoire a), on effectue la réaction d'un composé de formule générale III avec celui de formule générale IV en pyridine sous addition de triéthylamine à la température ambiante.

6. Procédé selon l'une des revendications 1 à 5, caractérisé en ce que dans le stade opératoire a), on cyclise un mélange d'isomères de formule générale V par addition d'un excès 1,5 à 3 fois molaire d'oxychlorure de phosphore à la température ambiante.

7. Procédé selon la revendication 1 ou 2 pour la préparation de la 2-(3-méthoxy-5-méthylsulfinyl-2-thiényl)-1H-imidazo(4,5-c)pyridine et de son chlorhydrate, caractérisé en ce qu'on traite la 2-(3-méthoxy-5-méthylthio-2-thiényl)-1H-imidazo(4,5-c)pyridine avec un peracide organique ou avec de l'eau oxygénée et, si on le désire, on transforme une base libre obtenue ou un sel pharmaceutiquement non acceptable de celle-ci en chlorhydrate.

8. Procédé selon l'une des revendications 1, 2 ou 7, caractérisé en ce qu'on effectue l'oxydation dans le stade b) avec des quantités sensiblement équivalentes d'acide 3-chloroperbenzoïque, d'acide perbenzoïque ou d'acide peracétique.

9. Procédé selon la revendication 8, caractérisé en ce qu'on effectue l'oxydation en chlorure de méthylène ou en chloroforme à −5°C jusqu'à −50°C, de préférence à environ 0°C.

10. Procédé selon les revendications 1, 2 ou 7, caractérisé en ce qu'on effectue l'oxydation avec des quantités sensiblement équivalentes d'eau oxygénée.

11. Procédé selon la revendication 10, caractérisé en ce qu'on effectue l'oxydation dans l'eau oxygénée à 30% en acide acétique à la température ambiante.

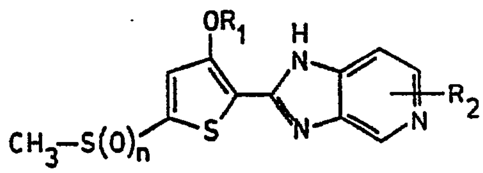

I

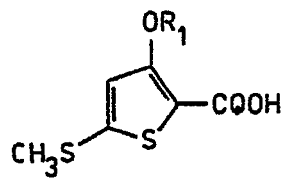

II

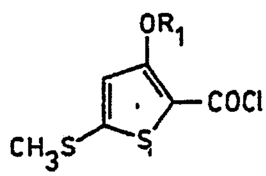

III

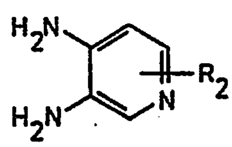

IV

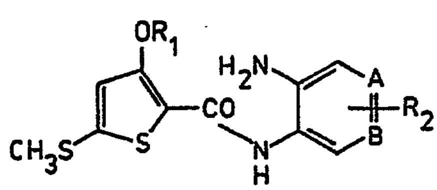

V